# FASCICULE DE BREVET EUROPEEN

(11) **EP 1 447 019 B1**
(45) Date de publication et mention de la délivrance du brevet: **31.05.2006**
(21) Numéro de dépôt: 04003330.0
(22) Date de dépôt: 14.02.2004
(51) Int. Cl.: A43B 5/00, A43B 13/12, A43B 13/14, A43C 15/09

(54) **Semelage de chaussure**
Schuhsohle
Shoe sole

(30) Priorité: 14.02.2003 FR 0301899
(43) Date de publication de la demande: 18.08.2004
(73) Titulaire: Salomon S.A., 74370 Metz-Tessy (FR)
(72) Inventeur: Mathieu, Guillaume, 38138 Les Cotes D'Arey (FR); Challe, Jean-Michel, 74150 Rumilly (FR)

(56) Documents cités:
- DE-C- 10 107 824
- US-A- 2 548 308
- US-A- 4 342 158
- US-A- 4 372 058
- US-A- 5 224 280
- US-A- 5 337 492

## Description

La présente invention concerne une chaussure destinée à la marche ou à la course notamment en montagne et plus particulièrement une semelle ou semelage conçue pour une telle chaussure.

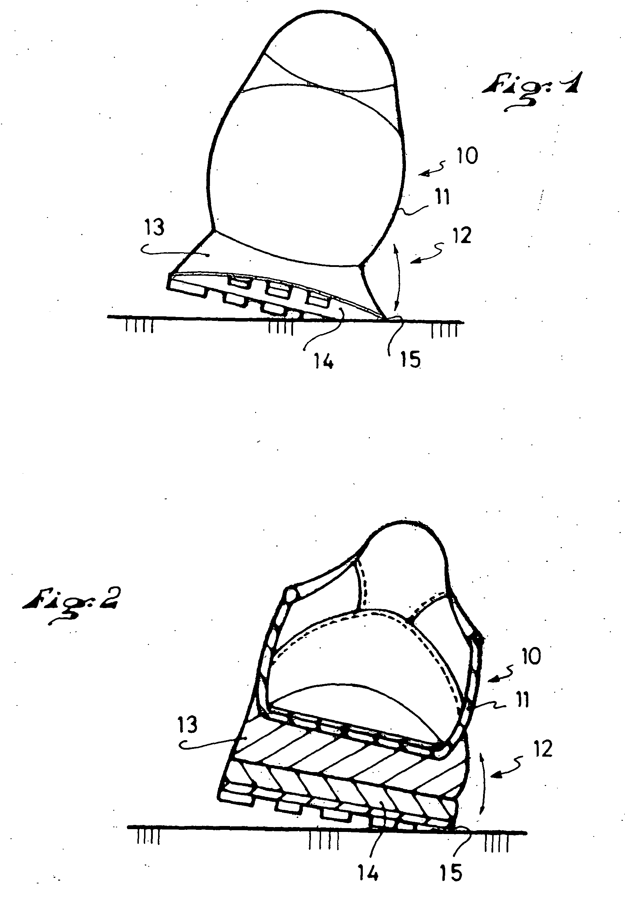

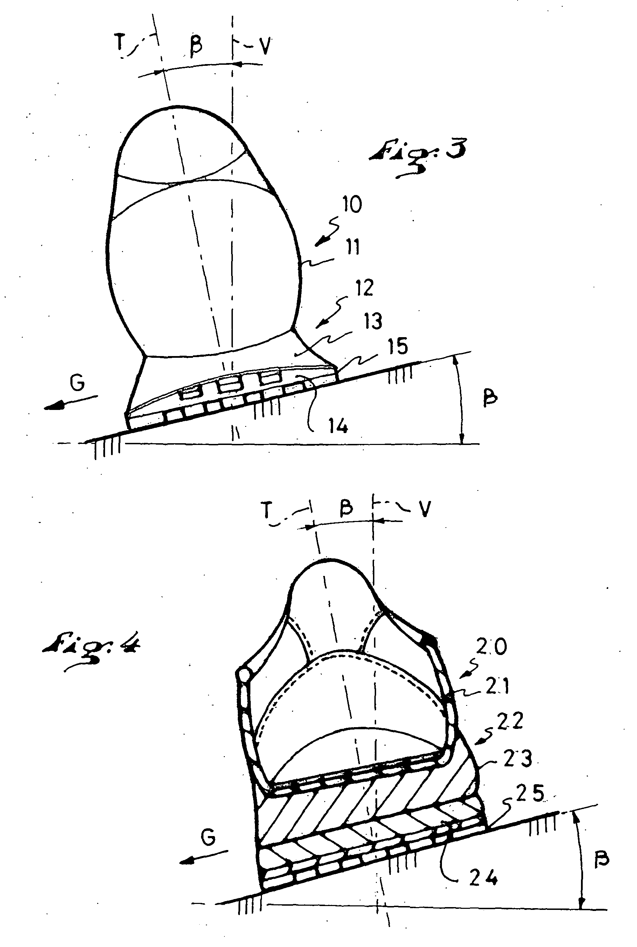

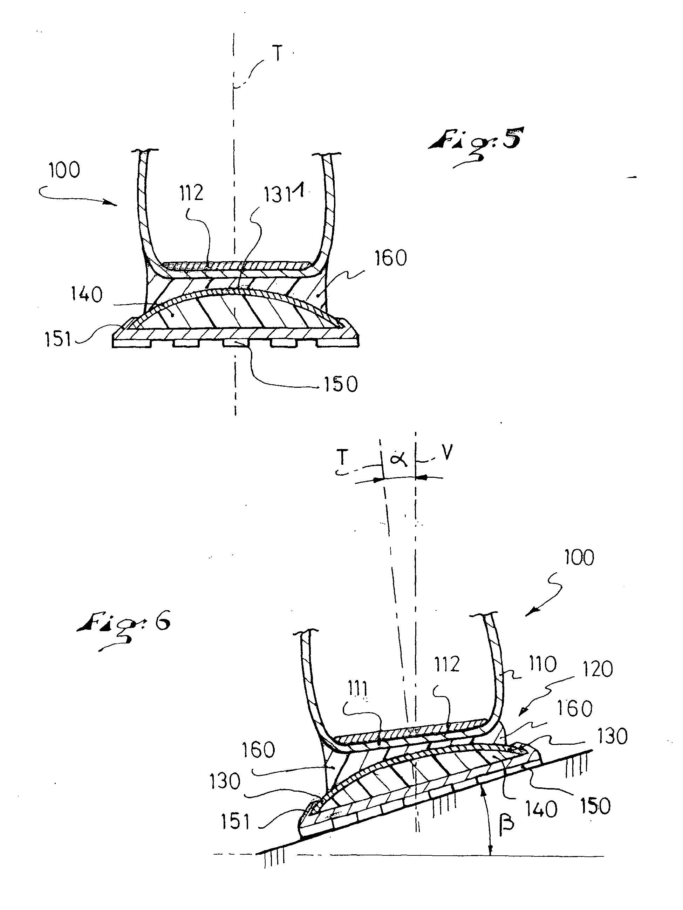

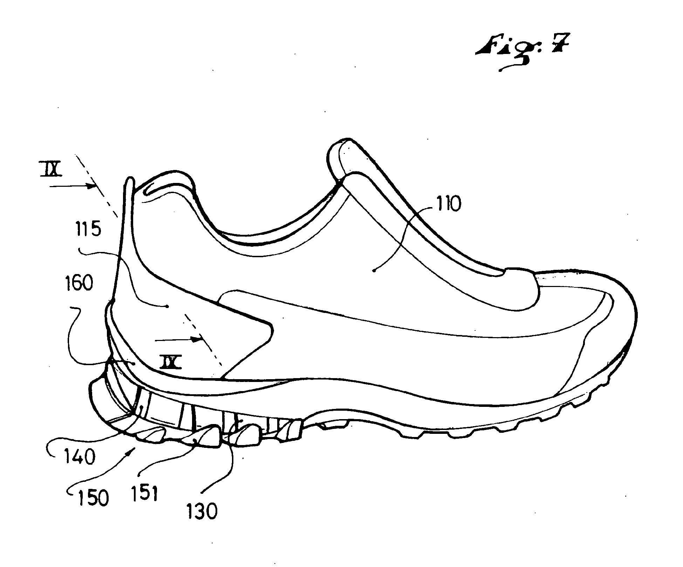

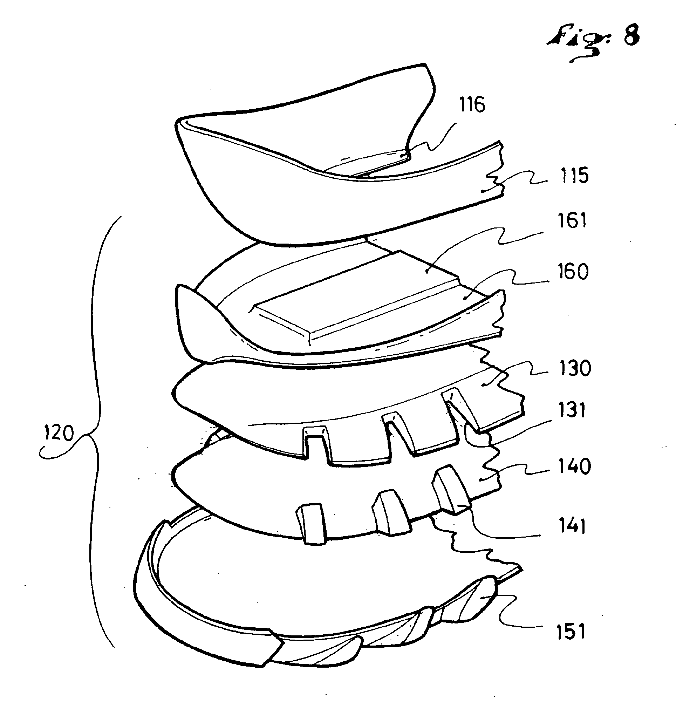

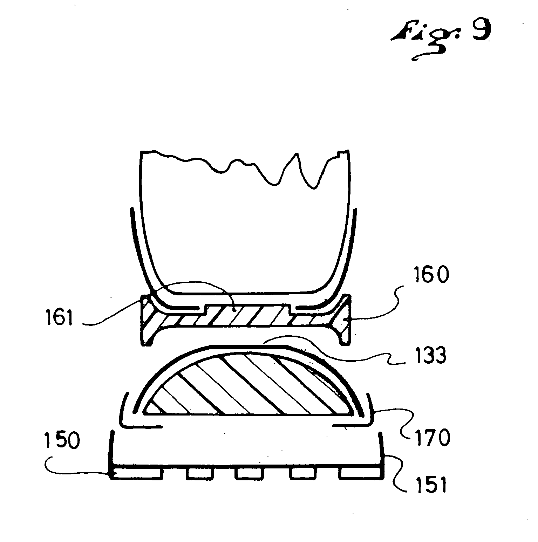

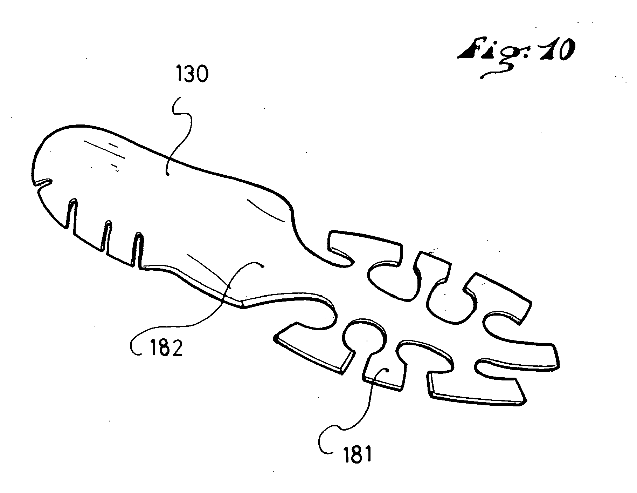

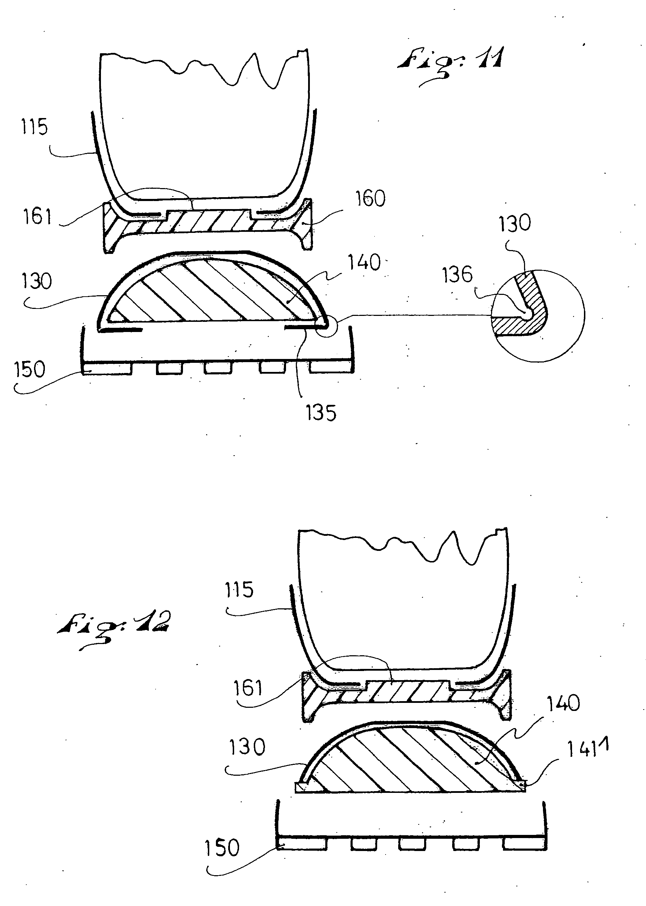

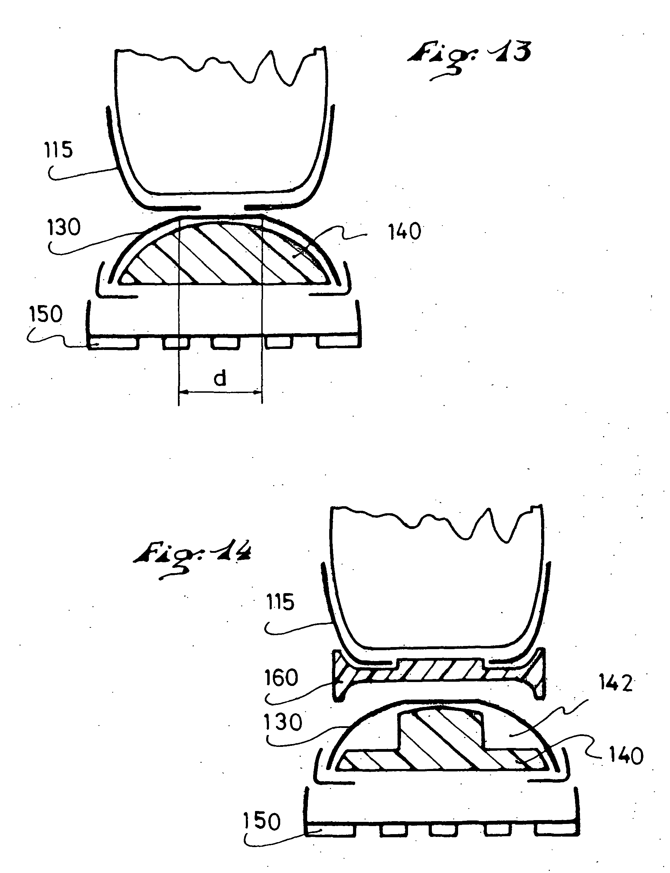

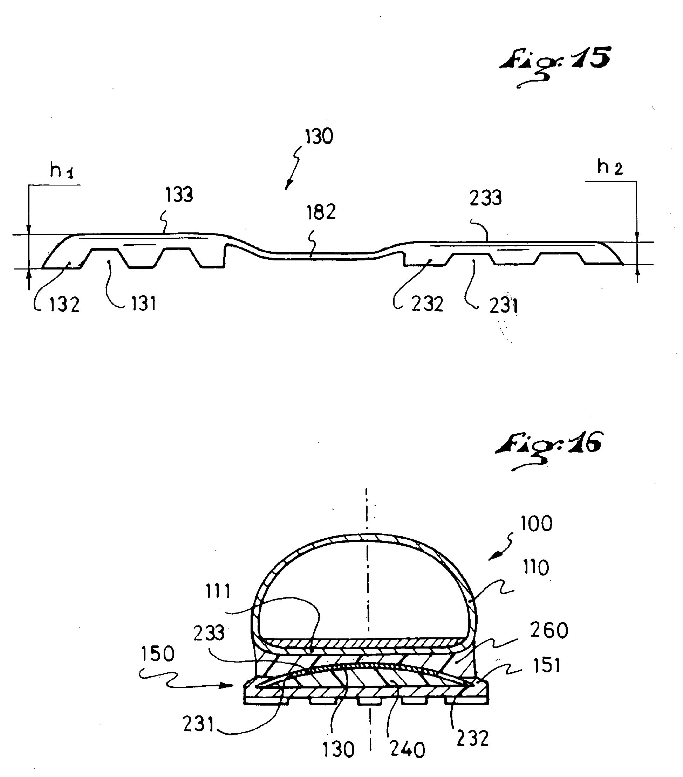

Les figures 1 à 4 illustrent les problèmes liés à l'utilisation de chaussures de course traditionnelles notamment en montagne.

Tout d'abord les chaussures de course à pied sont conçues généralement avec des moyens d'amortissement, notamment au niveau du talon, pour amortir les chocs répétés se produisant lors de la foulée ou aux endroits qui sont les plus sollicités par les chocs, et éviter les microtraumatismes sur les articulations de l'utilisateur.

Typiquement ainsi que le montre la figure 1, une telle chaussure 10 comporte une tige 11 montée sur un semelage 12, lequel semelage comporte une semelle intermédiaire 13 en matériau amortissant et une semelle de marche 14. Le semelage 12 a une forme, vue en coupe transversale, sensiblement trapézoïdale avec un bord 15 en forme d'arête vive. De ce fait, lors d'une flexion latérale ou médiale du pied ou de la jambe, la semelle intermédiaire 13 absorbera en partie les efforts supplémentaires en se comprimant.

Une fois cette semelle intermédiaire 13 complètement comprimée, la chaussure aura tendance à basculer brutalement par rapport à son bord 15, et peut alors provoquer des blessures (entorse,...etc.).

La figure 2 illustre un autre type de chaussure connue 20 comportant de même que la précédente une tige 21, un semelage 22 comportant une semelle intermédiaire 23 amortissante et une semelle de marche 24.

Dans ce second type de chaussure, décrit dans le US 4,322,895, on vise à éviter les problèmes évoqués ci-avant de basculement de la chaussure en faisant remonter la semelle intermédiaire le long de la tige. Cependant, ce second type de chaussure présente le même inconvénient de basculement brutal une fois la couche de la semelle intermédiaire 23 totalement comprimée. Dans le US 5 224 280 un élément élastiquement déformable est prévu au niveau du talon dans le semelage externe, mais de par sa forme il ne peut éviter le problème de basculement. Le US 5 337 492 comporte un profilé longitudinal de forme très complexe.

Par ailleurs, les chaussures de course à pied sont en général conçues pour coopérer avec des sols plats sur lesquels se déroulent en général les épreuves. Or, le développement de compétitions sportives de type "raid", incluant différentes activités sportives se déroulant dans un contexte montagneux, et incluant notamment des courses en montagne, impliquent de nouvelles contraintes sur les chaussures et utilisateurs. En effet, les courses en montagne se déroulent généralement sur des sols accidentés, en pente, non "plans", c'est-à-dire comportant de nombreuses aspérités, rocailles, et pouvant même présenter des dévers, c'est-à-dire des pentes transversales par rapport à l'axe de la course.

Comme il n'existe actuellement sur le marché que peu de chaussures de course réellement prévues pour de telles conditions, il s'ensuit pour les coureurs de nombreux problèmes traumatiques et risques d'accidents.

Les figures 3 et 4 illustrent le comportement des chaussures de type classique représentées en figure 1 et 2 sur des terrains en pente et notamment en dévers, c'est-à-dire comportant une pente en direction transversale par rapport à l'axe de la course.

Dans chacun de ces cas le semelage respectivement 12, 22 de chaque chaussure respectivement 10, 20 se déforme légèrement en fonction de la pente du terrain, mais de façon insuffisante de sorte que l'axe vertical médian T de la tige reste très incliné par rapport à la verticale V du lieu, et que la chaussure a tendance à glisser selon une direction G le long de la pente.

Au final, l'angle β que fait l'axe vertical médian de la tige avec la verticale V correspond à l'angle de dévers de la pente.

Le but de la présente invention est de remédier à ces inconvénients et de fournir une chaussure notamment de course comportant un semelage adapté permettant d'améliorer l'accroche de la chaussure sur terrain accidenté, en pente, en dévers, et permettant également une meilleure adaptation aux accidents et inégalités de terrain.

Un autre but de la présente invention est également de fournir une chaussure plus stable.

Enfin la chaussure selon l'invention doit présenter des caractéristiques d'amortissement compatibles avec l'utilisation en course.

Ce but est atteint dans la chaussure selon l'invention par les caractéristiques de la revendication 1.

En effet, l'élément élastique déformable en forme d'arche ou de voûte permet de reporter les efforts de l'utilisateur directement jusqu'au bord respectivement médial, latéral de la semelle externe, et donc d'augmenter notablement l'effet d'accroche par rapport à une chaussure de type traditionnel, où les efforts sont uniformément transmis, ceci même sur terrain en pente.

Par ailleurs, la capacité à se déformer de l'élément élastiquement déformable permet au semelage de se déformer de façon progressive et continue, en cas de flexion médiale ou latérale, et évite tout risque de basculement brutal qui pourrait provoquer des blessures (entorses,...etc.).

La réalisation de pattes ou branches indépendantes permet encore une meilleure adaptabilité de l'élément élastiquement déformable au terrain et aux différentes aspérités de celui-ci et permet donc de garantir une stabilité optimale de l'ensemble de la chaussure quel que soit le type de terrain.

De toute façon, l'invention sera mieux comprise et d'autres caractéristiques de celle-ci seront mises en évidence à l'aide de la description qui suit en référence au dessin schématique annexé en illustrant à titres d'exemples non limitatifs plusieurs modes de réalisation et dans lequel :
- les figures 1 et 2 sont des vues schématiques illustrant le comportement de chaussures de type connu en cas de flexion latérale,
- les figures 3 et 4 sont des vues similaires aux figures 1 et 2 illustrant le comportement de chaussures de type connu sur terrain en pente,
- la figure 5 est une vue en coupe transversale d'un premier mode de réalisation de l'invention,
- la figure 6 est une vue similaire à la figure 5 illustrant le fonctionnement de la chaussure sur terrain en pente,
- la figure 7 est une vue en perspective arrière d'une chaussure selon un second mode de réalisation,
- la figure 8 est une vue en perspective éclatée arrière de la partie talon de la chaussure de la figure 7,
- la figure 9 est une vue schématique en coupe selon IX-IX de la figure 7,
- la figure 10 est une vue en perspective d'un élément de semelage selon l'invention,
- la figure 11 est une vue schématique similaire à la figure 9 d'un troisième mode de réalisation,
- la figure 12 est une vue schématique similaire à la figure 11 d'un quatrième mode de réalisation,
- la figure 13 est une vue schématique similaire à la figure 11 d'un cinquième mode de réalisation,
- la figure 14 est une vue schématique similaire à la figure 11 d'un sixième mode de réalisation,
- la figure 15 est une vue en élévation d'un élément de semelage selon un autre mode de réalisation,
- la figure 16 est une vue en coupe transversale du semelage selon un autre mode de réalisation incorporant l'élément de semelage selon la figure 15.

Les figures 5 et 6 illustrent, par une coupe transversale schématique au niveau du talon, un premier mode de réalisation d'une chaussure 100 selon l'invention. Cette chaussure 100 comporte une tige 110 munie d'une semelle interne ou première de propreté 112 et un semelage 120.

Le semelage 120 comporte depuis le haut vers le bas :
- une cale de liaison 160 avec la tige 110,
- un élément élastiquement déformable 130 ayant en coupe transversale sensiblement la forme d'une voûte ou arche,
- une couche de matériau amortissant 140,
- une semelle externe ou de marche 150.

L'élément élastiquement déformable 130 est en un matériau relativement rigide mais élastiquement déformable et ayant un module d'Young E supérieur à 40 MPa.

Les matériaux préférés pour cet élément sont :
- Polyuréthane (PUR, TPU), chargé ou non chargé, de module d'Young E supérieur à 40 MPa.
- Polyamide (PA) chargé ou non chargé.
- Polyéthylène (PE) et globalement tous les matériaux synthétiques ayant un module d'Young E supérieur à 40 MPa.

Les matériaux "composites" ayant un module d'Young E supérieur à 50 MPa sont également envisageables.

Bien évidemment, l'épaisseur de l'élément élastique 130 sera fonction du degré d'élasticité souhaité et du module d'Young du matériau choisi.

Dans le cas représenté sur les figures 5 et 6, l'élément élastiquement déformable 130 a la forme d'une voûte régulière en portion d'arc de cercle, s'étendant depuis l'extrémité inférieure 111 de la tige 110 jusqu'aux bords respectivement médial et latéral 151 de la semelle externe 150.

Du fait de sa forme de voûte, une cale de 160 est nécessaire pour assurer la liaison de l'extrémité supérieure, arrondie, 1311 de l'élément élastique déformable 130 avec l'extrémité inférieure 111 de la tige. Cette cale 160 a, en coupe transversale, un bord supérieur 161 épousant la forme extérieure de la tige 110 et un bord inférieur 162 épousant la forme extérieure de l'élément élastiquement déformable 130.

La cale 160 peut être en un matériau de type mousse EVA, TPU, ou matériau compound ayant une dureté comprise entre 20 Asker C et 200 Asker C, de façon à procurer un effet d'amortissement supplémentaire et donc plus de confort au niveau du talon. Elle peut être également dans un autre matériau tel que PU, PA n'ayant pas nécessairement de qualité amortissante.

L'assemblage tige 110 / cale 160 / élément élastique 130 est effectué de façon connue en soi à l'aide de colles traditionnellement utilisées pour l'assemblage de semelles.

La couche de matériau amortissant 140 est, de même que la cale 160, en mousse EVA, TPU ou compound ayant une dureté comprise entre 20 et 200 Asker C.

Cette couche 140 est complètement emprisonnée entre l'élément élastique 130 et la semelle externe 150. Selon le mode de réalisation montré sur ces figures, les bords 150 de la semelle externe remontent légèrement sur ledit élément élastique 130.

Ainsi qu'on le concevra aisément et comme le montre la comparaison des figures 5 et 6, l'élément élastiquement déformable ou élément élastique 130 permet de transférer les efforts, appliqués centralement par le pied de l'utilisateur en haut de l'arche, sur les bords 150 de la semelle externe. De ce fait, l'effet d'accroche du semelage sur le terrain est considérablement augmenté, même sur un terrain accidenté et ayant une pente en dévers. Par ailleurs, cette transmission d'efforts s'accompagne d'une déformation élastique de l'élément élastique 130, permettant de redresser l'axe vertical médian T de la tige 110 et de l'amener au plus près de la verticale V du lieu, l'angle α étant donc inférieur à l'angle β.

Ce redressement de la tige 110 permet également de garantir une bonne stabilité du pied. Par ailleurs, du fait de sa force, l'élément élastique 130 peut se déformer de façon progressive et continue en s'aplatissant, et on évite les risques de basculement se produisant avec les chaussures de type connu.

Enfin, cette capacité du semelage à se déformer progressivement permet à l'utilisateur une bonne proprioception, et est une garantie supplémentaire pour limiter les risques de blessure.

La couche supplémentaire de matériau amortissant 140 permet, à hauteur égale de semelle, d'avoir un amortissement supplémentaire et donc plus efficace. En d'autres termes, pour une même efficacité d'amortissement il est possible de réduire la hauteur globale du semelage et donc d'augmenter encore la stabilité de la chaussure.

Les figures 7, 8, 9, 10 illustrant un second mode de réalisation de l'invention dans lequel les mêmes éléments seront désignés par les mêmes références.

Les figures 7 et 9 montrent particulièrement l'empilage des différentes couches du semelage dans la zone du talon, à savoir :
- semelle externe 150,
- matériau amortissant 140,
- élément déformable élastiquement 130,
- cale de liaison 160.

Par ailleurs dans ce mode de réalisation, la tige 110 est munie d'un contrefort talon externe 115 destiné à procurer plus de stabilité au pied et mieux transmettre l'effort du pied vers le sol via l'élément élastiquement déformable 130. Ce contrefort talon 115 est de préférence en un matériau synthétique ou composite rigide, et est choisi de façon à avoir un module d'Young E supérieur à 40 MPa. Il est assemblé à la tige 110 soit au moment de la mise en place du semelage 120, soit avant. Ce contrefort 115 peut être évidé comme représenté sur la figure 9, c'est-à-dire entourant la périphérie de la tige avec un rebord 116 vers l'intérieur, ou être muni d'un fond (non représenté) qui s'interpose alors entre ladite tige 110 et le semelage 120.

Bien entendu d'autres matériaux peuvent être prévus pour le contrefort.

L'élément élastique 130 est, muni de fentes latérales 131 délimitant des branches 132 s'étendant depuis le haut jusqu'en bas, sur les côtés du semelage, et aptes à se déformer élastiquement indépendamment les unes des autres.

Ces branches 132 permettent, d'une part, une plus grande élasticité générale de l'élément élastique 130, et d'autre part, une meilleure adaptation aux irrégularités du terrain du fait de leur capacité à se déformer indépendamment les uns des autres. Dans ce cas l'élément amortisseur 140 comporte des saillies 141 destinées à s'engager dans les fentes 131 et permettre un meilleur emboîtement avant l'assemblage final. L'élément élastique 130 présente également une zone supérieure 133 aplatie pour faciliter son assemblage sur la tige 110. La cale de liaison 160 présente également à sa partie supérieure une saillie 161 destinée à faciliter son emboîtement dans le contrefort de la tige 115 (cf. notamment figure 9).

Les bords 151 de la semelle de marche sont relevés et recouvrent en partie les extrémités inférieures de l'élément élastique 130 et de ses branches 132. Le cas échéant des morceaux de textile 170 peuvent être prévus entre l'élément élastique 130 et la semelle de marche 150 pour faciliter le collage à cette dernière.

Enfin, l'élément élastique 130 peut faire partie d'un élément de renfort de semelle 180 s'étendant jusqu'à l'avant du semelage. Dans ce cas, la partie avant 181 du renfort 180 est plane et se raccorde à la partie arrière 130 par une zone inclinée 182 au niveau de la zone de voûte plantaire.

Dans un mode de réalisation, la partie avant 181 du renfort 180 est directement en contact avec la semelle de marche de façon à procurer une meilleure accroche comme décrit dans le brevet US 6,079,125 au nom de la demanderesse.

Les figures 11 à 14 illustrent d'autres modes de réalisation pour lesquels les mêmes références numériques seront également utilisées pour désigner des éléments similaires ou identiques.

Dans le cas représenté à la figure 11, l'élément élastique 130 comporte à sa partie inférieure des retours 135 destinés à faciliter son collage sur la semelle externe 150. Ces retours 135 sont de préférence issus de moulage avec l'élément 130, une zone charnière 136 permettant de les replier après démoulage.

Dans le cas représenté à la figure 12, l'élément amortisseur 140 présente un rebord périphérique [1411] destiné à recevoir les extrémités inférieures de l'élément élastique 130 et faciliter l'assemblage du semelage 120.

Le mode de réalisation de la figure 13 correspond sensiblement à celui de la figure 9, la différence étant la suppression de la cale de liaison 160. Dans ce cas la zone plane supérieure 133 de l'élément élastique sera plus grande pour permettre un meilleur collage avec la tige. En règle générale cette zone plane 133 aura une largeur "d", en direction transversale, comprise entre 15 et 20mm.

Enfin, dans le dernier mode de réalisation de la figure 14, l'élément amortisseur présente des évidements <142> pour faciliter la déformation de l'élément élastique déformable 130.

Bien entendu, ces évidements <142> peuvent revêtir différentes formes, être en gradins, être asymétriques,... etc. L'important est que ces évidements <142> facilitent la déformation de l'élément élastiquement déformable 130.

Dans le mode de réalisation montré sur les figures 15 et 16, l'élément élastiquement déformable 130 a une forme de voûte non seulement à l'arrière dans la zone talon, mais également à l'avant dans la zone d'avant pied.

En ce qui concerne l'arrière les éléments similaires ou identiques seront désignés par les mêmes références.

A l'arrière l'élément élastiquement déformable 130 comporte donc une zone supérieure aplatie 133 s'étendant vers le bas par des branches 132 séparées par des fentes 131.

La zone supérieure aplatie 133 a comme le montre la figure 15 une hauteur h1 donnée qui est fonction du degré d'amortissement souhaité.

A l'avant l'élément élastiquement déformable 130 comporte une zone supérieure plus ou moins aplatie 233 qui s'étend vers le bas par des branches 232 séparées par des fentes 231.

Comme le montre la figure 15 la zone supérieure aplatie 233 de l'avant pied a une hauteur h2 qui sera généralement plus basse que la hauteur h1. Bien entendu, de même que précédemment, la hauteur h2, sera fonction de l'amortissement souhaité.

Selon les effets désirés (par exemple musculation de la jambe) h2 pourrait également être au contraire supérieure à h1.

Une zone de transition 182 sépare les deux parties 133, 233 de l'élément élastiquement déformable 130.

La figure 16 montre l'incorporation de la partie 233 de l'élément élastiquement déformable 130 dans la partie avant pied d'un semelage.

Dans ce cas, l'élément élastiquement déformable 130 a également dans la zone d'avant pied sensiblement la forme transversale d'une arche s'étendant vers le bas depuis l'extrémité inférieure 111 de la tige 110 jusqu'aux bords respectivement médial et latéral de la semelle externe 150.

La figure 16 montre bien l'empilage des différentes couches du semelage dans la zone avant pied, à savoir du bas vers le haut :
- semelle externe 150,
- matériau amortissant 240,
- élément élastiquement déformable 130,
- cale de liaison 260.

De même que décrit précédemment, les bords 151 sont dans ce mode de réalisation relevés et recouvrent en partie les extrémités inférieures de l'élément élastique 130 et de ses branches 232.

Bien entendu, le fonctionnement est le même que décrit précédemment, à savoir que l'élément élastique 130 permet de transférer les efforts, appliqués centralement par le pied de l'utilisateur en haut de l'arche, sur les bords 150 de la semelle externe. De ce fait l'effet d'accroche du semelage sur le terrain est considérablement augmenté, aussi bien à l'avant qu'à l'arrière de la chaussure.

Selon le type de chaussure et d'application, l'effet d'accroche ci-dessus peut être prévu uniquement à l'avant, uniquement à l'arrière ou dans les deux zones en même temps.

Bien entendu, la présente invention n'est pas limitée aux modes de réalisation décrits ci-avant à titre d'exemples non limitatifs, mais en englobe tous les modes de réalisation compris dans la portée des revendications qui suivent.

## Revendications

1. Chaussure comportant une tige (110) et un semelage externe (120), le semelage externe comprenant un élément élastiquement déformable (130) et une semelle externe ou de marche (150)
l'élément élastique déformable (130) étant disposé dans la zone du talon et/ou de l'avant pied et ayant en direction transversale sensiblement la forme d'une arche s'étendant vers le bas depuis l'extrémité inférieure (111) de la tige (110), jusqu'aux bords (151) respectivement médial et latéral de la semelle externe (150) le semelage comportant également une couche (140) de matériau amortissant complètement emprisonnée entre l'élément élastique et la semelle externe (150) **caractérisés en ce que** l'élément élastique (130) est muni de fentes latérales (131, 231) délimitant des branches (132, 232) s'étendant sur les côtés du semelage.

2. Chaussure selon la revendication 1, **caractérisée en ce que** l'élément élastiquement déformable (130) est en un matériau ayant un module d'Young d'au moins 40 MPa.

3. Chaussure selon la revendication 1 ou 2, **caractérisée en ce que** l'élément élastiquement déformable (130) est en polyuréthane, chargé ou non, polyamide chargé ou non, polyéthylène.

4. Chaussure selon l'une des revendications 1 à 3, **caractérisée en ce que** l'élément élastiquement déformable (130) est en un matériau composite ayant un module d'Young supérieur à 50 MPa.

5. Chaussure selon l'une des revendications 1 à 4, **caractérisée en ce que** l'élément élastiquement déformable (130) est muni à son extrémité supérieure d'une zone sensiblement plane (133, 233).

6. Chaussure selon la revendication 5, **caractérisée en ce que** la zone plane (133, 233) a une largeur d'environ 15 à 20 mm.

7. Chaussure selon l'une des revendication 1 à 6, **caractérisée en ce que** l'élément élastiquement déformable (130) comporte de chaque côté des branches (132, 232) respectivement médiales, latérales.

8. Chaussure selon l'une des revendications 1 à 7, **caractérisée en ce que** l'élément élastique (130) est disposé dans la zone talon.

9. Chaussure selon l'une des revendications 1 à 8, **caractérisée en ce que** l'élément élastique est disposé dans la zone avant.

10. Chaussure selon les revendications 8 et 9, **caractérisée en ce que** l'élément élastique a une hauteur (h2) dans la zone avant pied, plus basse que sa hauteur (h1) dans la zone talon.

11. Chaussure selon les revendications 8 et 9 **caractérisée en ce que** l'élément élastique a dans la zone avant pied une hauteur (h2) supérieure à celle (h1) de la zone talon.

12. Chaussure selon la revendication 8, **caractérisée en ce que** l'élément élastique s'étend jusqu'à l'avant du semelage par une partie avant plane (181).

13. Chaussure selon la revendication 12, **caractérisée en ce que** la partie avant (181) se raccorde à la partie arrière (130) par une zone inclinée (182) au niveau de la voûte plantaire.

14. Chaussure selon la revendication l'une des revendications 1 à 13, **caractérisée en ce que** la couche (140) de matériau amortissant comporte au moins un évidement (142) entre ladite couche et l'élément élastiquement déformable (130).

15. Chaussure selon l'une des revendications 1 à 14, **caractérisée en ce que** l'élément élastiquement déformable (130) est fixé à la tige via une cale (160, 260).

16. Chaussure selon la revendication 15, **caractérisée en ce que** la cale (160, 260) est en matériau de type EVA, TPU.

17. Chaussure selon la revendication 15 **caractérisée en ce que** la cale (160, 260) est en matériau tel que PU, PA.

18. Chaussure selon l'une des revendications 1 à 17, **caractérisée en ce qu'**un contrefort extérieur (115) est interposé entre la tige (110) de la chaussure et l'élément élastiquement déformable (130).

19. Chaussure selon la revendication 18, **caractérisée en ce que** le contrefort (115) est en un matériau ayant un module d'Young supérieur à 40 MPa.

## Claims

1. Shoe comprising an upper (110) and an outer bottom assembly (120), the outer bottom assembly comprising an elastically deformable element (130) and an outsole or walking sole (150), the elastically deformable element (130) being arranged in the heel and/or forefoot zone and substantially having, in the transverse direction, an arch shape extending downward from the lower end (111) of the upper (110), to the medial and lateral edges (151), respectively, of the outsole (150), the bottom assembly also comprising a layer (140) of shock-absorbing material confined between the elastic element and the outsole (150), **characterized in that** the elastic element (130) is provided with lateral slits (131, 231) demarcating arms (132, 232) extending over the sides of the bottom assembly edges.

2. Shoe according to claim 1, **characterized in that** the elastically deformable element (130) is made of a material having a Young's modulus of at least 40 Mpa.

3. Shoe according to claim 1 or 2, **characterized in that** the elastically deformable element (130) is made of polyurethane, reinforced or not reinforced, polyamide, reinforced or not reinforced, polyethylene.

4. Shoe according to one of claims 1 to 3, **characterized in that** the elastically deformable element (130) is made of a composite material having a Young's modulus greater than 50 Mpa.

5. Shoe according to one of claims 1 to 4, **characterized in that** the elastically deformable element (130) is provided at its higher end with a substantially planar zone (133, 233).

6. Shoe according to claim 5, **characterized in that** the planar zone (133, 233) has a width of about 15-20 mm.

7. Shoe according one of claims 1 to 6, **characterized in that** the elastically deformable element (130) comprises on each side medial, lateral arms (132, 232), respectively.

8. Shoe according to one of claims 1 to 7, **characterized in that** the elastically deformable element is arranged in the heel zone.

9. Shoe according to one of claims 1 to 8, **characterized in that** the elastically deformable element is arranged in the forefoot zone.

10. Shoe according to claims 8 and 9, **characterized in that** the elastic element has a height (h2) in the forefoot zone lower than its height (h1) in the heel zone.

11. Shoe according to claims 8 and 9, **characterized in that** the elastic element has a height (h2) in the forefoot zone that is greater than that (h1) of the heel zone.

12. Shoe according to claim 8, **characterized in that** the elastic element extends up to the front of the bottom assembly by means of a planar front portion (181).

13. Shoe according to claim 12, **characterized in that** the front portion (181) connects to the rear portion (130) by means of an inclined zone (182) in the area of the arch of the foot.

14. Shoe according to one of claims 1 to 13, **characterized in that** the layer (140) of shock absorbing material comprises at least one recess (142) between said layer and the elastically deformable element (130).

15. Shoe according to one of claims 1 to 14, **characterized in that** the elastically deformable element (130) is fixed to the upper via a wedge (160, 260).

16. Shoe according to claim 15, **haracterized** that in that the wedge (160, 260) is made of a material of the EVA, TPU type.

17. Shoe according to claim 15, **characterized in that** the wedge (160, 260) is made of a material such as PU, PA.

18. Shoe according to one of claims 1 to 17, **characterized in** an outer stiffener (115) is inserted between the upper (110) of the shoe and the elastically deformable element (130).

19. Shoe according to claim 18, **characterized in that** the stiffener (115) is made of a material having a Young's modulus greater than 40 Mpa.

## Patentansprüche

1. Schuh, aufweisend einen Schaft (110) und einen äußeren Sohlenaufbau (120), wobei der äußere Sohlenaufbau ein elastisch verformbares Element (130) und eine äußere Sohle oder Laufsohle (150) aufweist, wobei das elastisch verformbare Element (130) in dem Bereich der Ferse und/oder des Vorderteils des Fußes angeordnet ist und in im Wesentlichen transversaler Richtung die Form eines Bogens aufweist, der sich in Richtung nach unten ausgehend von dem unteren Ende (111) des Schaftes (110) bis zu den Rändern (151), dem medialen und dem lateralen Rand der äußeren Sohle (150), erstreckt, wobei der Sohlenanfbau ebenso eine Schicht (140) aus einem vollständig dämpfenden Material aufweist, die zwischen dem elastischen Element und der äußeren Sohle (150) eingeschlossen ist, **dadurch gekennzeichnet, dass** das elastische Element (130) mit seitlichen Spalten (131, 231) versehen ist, welche Äste (132, 232) begrenzen, die sich auf den Seiten des Sohlenaufbaus erstrecken.

2. Schuh nach Anspruch 1, **dadurch gekennzeichnet, dass** das elastisch verformbare Element (130) aus einem Material besteht, welches ein Young-Modul von wenigstens 40 MPa aufweist.

3. Schuh nach Anspruch 1 oder 2, **dadurch gekennzeichnet, dass** das elastisch verformbare Element (130) aus verstärktem oder nicht-verstärktem Polyurethan besteht, verstärktem oder nicht-verstärktem Polyamid oder Polyethylen.

4. Schuh nach einem der Ansprüche 1 bis 3, **dadurch gekennzeichnet, dass** das elastisch verformbare Element (130) aus einem Verbundmaterial besteht, welches ein Young-Modul größer als 50 MPa aufweist.

5. Schuh nach einem der Ansprüche 1 bis 4, **dadurch gekennzeichnet, dass** das elastisch verformbare Element (130) an seinem oberen Ende mit einem im Wesentlichen ebenen Bereich (133, 233) versehen ist.

6. Schuh nach Anspruch 5, **dadurch gekennzeichnet, dass** der ebene Bereich (133, 233) eine Breite von in etwa 15 bis 20 mm aufweist.

7. Schuh nach einem der Ansprüche 1 bis 6, **dadurch gekennzeichnet, dass** das elastisch verformbare Element (130) auf jeder Seite jeweils mediale bzw. laterale Äste (132, 232) aufweist.

8. Schuh nach einem der Anspruche 1 bis 7, **dadurch gekennzeichnet, dass** das elastische Element (130) in dem Bereich der Ferse angeordnet ist.

9. Schuh nach einem der Ansprüche 1 bis 8, **dadurch gekennzeichnet, dass** das elastische Element in dem vorderen Bereich angeordnet ist.

10. Schuh nach den Ansprüchen 8 und 9, **dadurch gekennzeichnet, dass** das elastische Element in dem Bereich des Vorderteils des Fußes eine Höhe (h2) niedriger als seine Höhe (h1) in dem Bereich der Ferse aufweist.

11. Schuh nach den Ansprüchen 8 und 9, **dadurch gekennzeichnet, dass** das elastische Element in dem Bereich des Vorderteils des Fußes eine Höhe (h2) größer zu derjenigen (h1) des Fersenbereichs aufweist.

12. Schuh nach Anspruch 8, **dadurch gekennzeichnet, dass** das elastische Element sich bis zur Vorderseite des Sohlenaufbaus durch einen ebenen vorderen Teil (181) erstreckt.

13. Schuh nach Anspruch 12, **dadurch gekennzeichnet, dass** der vordere Teil (181) mit dem hinteren Teil (130) über eine geneigte Zone (182) auf Höhe des Fußgewölbes verbunden ist.

14. Schuh nach einem der Ansprüche 1 bis 13, **dadurch gekennzeichnet, dass** die Schicht (140) aus dämpfendem Material mindestens eine Aussparung (142) zwischen der Schicht und dem elastisch verformbaren Element (130) aufweist.

15. Schuh nach einem der Ansprüche 1 bis 14, **dadurch gekennzeichnet, dass** das elastisch verformbare Element (130) an dem Schaft über einen Keil (160, 260) befestigt ist.

16. Schuh nach Anspruch 15, **dadurch gekennzeichnet, dass** der Keil (160, 260) aus einem Material vom Typ EVA oder TPU besteht.

17. Schuh nach Anspruch 15, **dadurch gekennzeichnet**, class der Keil (160, 260) aus einem Material wie z.B. PU oder PA besteht.

18. Schuh nach einem der Ansprüche 1 bis 17, **dadurch gekennzeichnet, dass** eine äußere Fersenkappe (115) zwischen den Schaft (110) des Schuhs und das elastisch verformbare Element (130) gesetzt ist.

19. Schuh nach Anspruch 18, **dadurch gekennzeichnet, dass** die Fersenkappe (115) aus einem Material besteht, welches ein Young-Modul größer als 40 MPa aufweist.
